# EUROPEAN PATENT APPLICATION

(11) **EP 1 992 948 A1**
(43) Date of publication of application: **19.11.2008**
(21) Application number: 07108158.2
(22) Date of filing: 14.05.2007
(51) Int. Cl.: G01N 33/50, G01N 33/543

(54) **Biosensor device, method of manufacturing a biosensor device and method of detecting target molecules in an analyte**

(71) Applicant: Koninklijke Philips Electronics N.V., 5621 BA Eindhoven (NL); Philips Intellectual Property & Standards GmbH, 20099 Hamburg (DE)
(72) Inventor: The designation of the inventor has not yet been filed
(74) Representative: Van Velzen, Maaike Mathilde

(57) **Abstract**

The biosensor device (10) for detecting target molecules (12) in an analyte (14) comprises a substrate (18) which can be brought into contact with the analyte (14) for binding the target molecules (12) thereto. Capture molecules (36) for the target molecules (12) are immobilized in at least one defined area (30 a-k) of the substrate (18). The remaining area (32) of the substrate (18) is covered with a coating layer (26) which acts as a blocking agent for the target molecules (12).

## Description

### FIELD OF THE INVENTION

The invention relates to a biosensor device for detecting target molecules in an analyte.

The invention further relates to a method of manufacturing a biosensor device for detecting target molecules in an analyte.

The invention further relates to a method for detecting target molecules in an analyte.

### BACKGOUND OF THE INVENTION

Biosensor devices are generally known from US 2004/0152135 Al or US 2002/0102565 Al.

In general, a biosensor device comprises a substrate which can be brought into contact with an analyte for binding target molecules thereto. Upon binding to a two-dimensional or three-dimensional binding area of the substrate, the target molecules generate a signal which can be detected by a detection system for e.g. an identification of the target molecule sort and a qualitative or quantitative analysis of the target molecule concentration. The signal to be detected can be an optical signal such as light emitted from the target molecules upon excitation, or it can be a color change of the analyte resulting from a chemical reaction of the target molecules and the binding area surface. Other detection techniques are also encompassed by the present invention.

It is known, that the signal intensity and thus the sensitivity of the biosensor device is increased by providing a three-dimensional structure as binding area. This structure can be realized as e.g. porous body comprising channels. The porous body can be designed as a layer on the surface of the substrate, where the channels extend from the top surface of the layer into or through the layer. The binding area for the target molecules is thus enhanced without significantly expanding the volume of the biosensor device, since the target molecules can also bind to the wall surfaces of the channels.

It is further known, that capture molecules can be immobilized on the surface of the binding area for increasing the specificity of the biosensor device. The capture molecules act as specific recognition partners for the complementary target molecules to be detected, whereby unspecific bindings of e.g. impurity molecules to the binding area and thus background noise resulting from such unspecific binding events are significantly reduced.

The biosensor device known from US 2004/0152135 Al comprises a substrate made of a bulk semiconductor material such as silicon (Si). The substrate comprises at least one porous body having channels. The porous bodies are formed on the top surface of the bulk substrate, and they are made of the material of the substrate. Capture molecules for the target molecules are immobilized on the porous bodies which are separated from one another by the substrate. Binding the target molecules to the capture molecules results in a change of the refractive index of the substrate, which is detected as wavelength shift in a reflectometric interference spectrum of the substrate compared to a reference substrate without bound target molecules.

It is a disadvantage of the known biosensor device, that it shows a high level of background noise resulting from unspecific bindings of the target molecules to the substrate having no immobilized capture molecules. This effect even enhances in case of the material of the substrate showing an intrinsic affinity for the target molecules. Therefore the signal to noise ratio of the known biosensor device decreases, leading to a limitation of the sensitivity of the biosensor device.

It is a further disadvantage of the known biosensor device, that unspecific binding events and adsorption of molecules in those areas where no capture molecules are immobilized will blur the size of the defined areas of the porous bodies being relevant for the signal, which will complicate the data processing and which leads to a reduced sensitivity.

It is a further disadvantage of the known biosensor device, that the manufacturing process of the semiconductor substrate is highly complicated, since several porous bodies have to be etched in the surface of the substrate. Therefore the manufacturing costs of the known biosensor device are high.

It is further disadvantageous, that the measurement principle as well as the data analysis of the known biosensor device are highly complicated, since the measurement and analysis precession strongly depend on the properties of the reference substrate such as its surface roughness. Therefore high experience concerning the realization of the measurements and the data analysis of the measured spectra are demanded, resulting in a difficult handling of the biosensor device.

A further biosensor device is known from US 2002/0102565 Al mentioned above. The biosensor device comprises a metal oxide substrate in the form of a porous body with through-going channels. The surface of the substrate, i.e. the top surface of the substrate as well as the wall surfaces of the channels, are functionalized with capture molecules acting as recognition partner for the target molecules to be detected. Label molecules binding to the target molecules-capture molecules complexes create a signal in the form of a color change or bio-, chemo- or photoluminescent light.

It is a disadvantage of the known biosensor device that absorption of molecules and unspecific bindings of the target molecules to not-functionalized parts of the surface of the substrate increase the level of background noise of the biosensor device resulting in a decrease of its sensitivity.

### SUMMARY OF THE INVENTION

Therefore it is an object of the present invention to improve the biosensor device, the method of manufacturing the biosensor device and the method for detecting target molecules in an analyte of the kinds mentioned at the outset, so that a biosensor device is provided having a high detection sensitivity with a low background noise.

According to the invention, this object is achieved by a biosensor device comprising a substrate which can be brought into contact with the analyte for binding the target molecules thereto, wherein capture molecules for the target molecules are immobilized in at least one defined area of the substrate, wherein the remaining area of the substrate is covered with a coating layer which acts as a blocking agent for the target molecules.

Further, according to the invention, a method of manufacturing a biosensor device is provided, comprising the steps of (a) providing a substrate, (b) covering the substrate with a coating layer which acts as a blocking agent for the target molecules, (c) removing the coating layer from at least one defined area of the substrate, and (d) immobilizing capture molecules for the target molecules in the at least one defined area of the substrate.

Further, according to the invention, a method for detecting target molecules in an analyte is provided comprising the steps of (a) providing a biosensor device according to the invention, (b) binding target molecules to at least one defined area of a substrate of the biosensor device, and (c) detecting a signal generated by the target molecules upon binding to the at least one defined area.

The biosensor device, the method of manufacturing the biosensor device and the method of detecting target molecules in an analyte according to the invention make use of a substrate which can be brought into contact with the analyte for binding the target molecules thereto. The substrate forms a two-dimensional and/or a three-dimensional binding area for the target molecules. In addition, capture molecules acting as recognition partners for the complementary target molecules are immobilized on a surface of at least one defined area of the substrate. The remaining area of the substrate, that surrounds the defined area, is covered by a coating layer. The coating layer acts as a blocking agent for the target molecules, whereby a binding of the target molecules to the coating layer is prevented. The biosensor device thus advantageously offers a sensitive detection means for specific target molecules which simultaneously exhibits a low background signal. Unspecific binding events which result from bindings of the target molecules to the not functionalized remaining area are prevented by sealing the surface of the remaining area with the coating layer. Furthermore, this coating layer does not exhibit an intrinsic affinity for the target molecules to be detected, but rather shows a repulsion effect for the target molecules, so that no signal will be generated by the target molecules binding to the surface of the remaining area. Consequently, the signal to noise ratio of the biosensor device is significantly enhanced.

Furthermore, it is an advantage, that the binding probability of the target molecules to the capture molecules is increased compared to the known biosensor device having no coating layer on the remaining area, as only the functionalized surface of the defined area is accessible for the target molecules. Therefore the target molecules can only come into contact with those parts of the surface comprising the immobilized capture molecules, so that the signal intensity of the biosensor device and thus its sensitivity is increased.

It is a further advantage, that the coating layer offers the possibility of defining the shape of the reactive defined area being relevant for the signal to be detected, as the coating layer borders the defined area. The defined area can be shaped in an e.g. round or rectangular way depending on the size of the substrate.

Referring to the method for manufacturing the biosensor device according to the invention, the substrate is first totally covered with the coating layer. This coating layer is then removed from the at least one defined area, which is subsequently functionalized with the capture molecules. This particular sequence of the manufacturing steps of the biosensor device advantageously provides the easiest way of creating a defined area which comprises the immobilized capture molecules and is surrounded by the remaining area of the substrate having no immobilized capture molecules and being coating with a blocking agent for the target molecules. Moreover, the production costs of the biosensor device in terms of the amount of the capture molecules can be significantly decreased.

In a preferred embodiment of the invention, the substrate comprises a porous body having channels, wherein the defined area comprises at least one of the channels.

This measure provides a particular embodiment of a three-dimensional binding area in the form of a porous body with channels. The porous body can be embedded within the substrate with the channels penetrating into the substrate or the substrate can be formed as porous body with through-going channels. The capture molecules are immobilized on the surface of the substrate within the defined area. Here, the expression "surface" refers to the top and/or lower surfaces of the porous substrate as well as to the wall surfaces of the channels. The coating layer covers the remaining surface of the porous substrate. In this context, the expression "covering" the remaining area of the porous substrate refers to a coating of the top and/or lower surfaces of the remaining area as well as to an at least partial coating of the wall surfaces of the channels of the remaining area. As the defined area represents a three-dimensional structure, the binding area for the target molecules as well as the binding kinetics are advantageously increased, which enhances the signal of the biosensor device. Moreover, the biosensor device can be designed in a very compact way, since a sufficient signal can be obtained using a smaller binding area compared to a biosensor with a two-dimensional binding area.

It is a further advantage, that the manufacturing process of the porous substrate is simpler compared to the known biosensor device, since a substrate with only one porous body has to be provided instead of one comprising several porous bodies. The shape and the position of the defined area can be adapted by the coating layer, whereby the manufacturing process of the biosensor device is very flexible and inexpensive.

In a further preferred embodiment of the invention, the coating layer is a photosensitive material.

A photosensitive coating layer advantageously enables the coating step of the (porous) substrate to be easily performed, as an intrinsic property of the material of the coating layer can be used to support the coating process.

In a further preferred embodiment of the invention, the coating layer is made of a polymer.

In this context, using a polymer as coating layer material offers an inexpensive covering of the (porous) substrate whose properties such as hardness, smoothness, etc. can be influenced by the diverse polymer properties and thus adapted to the requirements of the biosensor device. In particular, polymers can provide a hard, smooth and stable layer on top of the (porous) substrate which does not comprise free bonds, whereby binding of the target molecules to it is prevented. Furthermore, handling of this coating material is very easy in the sense that polymer chemistry is sufficiently known in the prior art.

Preferably, the coating layer is a polymeric hydrogel based on e.g. acrylamide and bis-acrylamide or on methacrylate and dimethacrylate whose polymerization is initialized by photoinitiators such as water soluable thioxanthone and benzophenone. The hydrogel swells in water and retains a significant fraction of water within its structure without dissolving in water. It forms an equilibrium state with the water and maintains its equilibrium state. The pore size and thus the equilibrium content of water of the hydrogel can be adjusted by varying the ratio of the monomer and the cross linker co-monomer. In addition, hydrogels offers the advantage of being transparent for light and colorless.

In a further preferred embodiment of the invention, the coating layer is made of photo resist.

The advantage here is, that the photo resist which is also made of a polymer forms a very cheap material for covering the surface of the substrate. Furthermore, using a photo resist as coating material advantageously offers the user of the biosensor device the possibility to create himself the coating layer of the substrate within a short time by a light exposure process, as the photo resist can be easily handled and it instantaneously dries upon heating.

In a further preferred embodiment of the invention the coating layer is a solution sensitive material.

Using a coating layer with such a property advantageously allows a very simple and inexpensive dissolution of the coating layer at the defined area for removing the coating layer material from the defined area.

In a further preferred embodiment of the invention, the substrate and/or the porous body is made of a polymer.

The advantage here is, that the manufacturing of an especially porous substrate is very easy and inexpensive, as a polymer based material can be homogeneously designed with pores of an average pore size.

In a further preferred embodiment of the invention, the biosensor device comprises a detection system for detecting a signal generated by the target molecules upon binding to the at least one defined area.

This measure causes the detection system to only detect those signals resulting from binding events of target molecules to capture molecules, whereby background noise in terms of signal intensity resulting from unspecific binding events or from not bound target molecules are further suppressed.

In this context, it is to be understood, that the signal can be generated by the target molecules upon binding to the capture molecules which are immobilized in the defined area of the substrate. However, the signal can also be generated by label molecules bound to the target molecules, wherein the signal arises from the binding event of complexes formed by the target molecules and the label molecules to the capture molecules.

In the further preferred embodiment of the invention, the detection system is an optical detection system which is arranged next to the coating layer.

An optical detection system is based on the principle of first exciting the binding area by light and afterwards detecting the generated optical signal by an optical detection system. The signal arises from the binding events of the target molecules to the capture molecules. For instance, the signal can be fluorescent, luminescent or phosphorescent light emitted by the target molecules upon their excitation or any light scattered by the bound target molecules. An optical detection system advantageously provides an easily manageable detection mechanism for analyzing the optical signal in terms of a signal conversion into a quantitative target molecule concentration. Furthermore, arranging the detection system next to the coating layer advantageously offers the possibility to enhance the signal intensity, as absorption effects of the optical signal along the interface of the optical system and the substrate and at the substrate are avoided.

In this context, the polymeric coating layer reduces the surface roughness of the substrate by e.g. smoothing the unevennesses of the substrate and filling the pores of the substrate. This advantageously further decreases the background signal of the biosensor device which results from light scattering at the surface of the binding area.

In a further preferred embodiment of the invention, a refractive index of the coating layer is adapted to a refractive index of the analyte.

This measure has the advantage, that the background noise of the biosensor device which results from light scattering, in particular light reflection, at the interfaces analyte-coating layer and coating layer-substrate is significantly reduced, since the refractive indices of the analyte and the substrate can be adjusted by the refractive index of the coating layer. The coating layer comprises an intermediate refractive index compared to the refractive indices of the analyte and the substrate, wherein the refractive index of the polymeric coating layer can be modified by its ratio of monomers and co-monomers. In case of a polymeric coating layer, e.g. the hydrogel, the refractive index of the coating layer can be varied e.g. between 1.3 and 1.5 by changing its structure and its equilibrium water content. Preferably, the refractive index of the coating layer is 1.49.

In a further preferred embodiment of the invention, the coating layer comprises colorants acting as optical filters.

Here, polymerizable and non-polymerizable colorants such as benzophenones in the form of a layer on or in the coating layer or in the form of single pigments are integrated into the coating layer. These colorants act as optical filters and absorb light of particular wavelengths, whereby unintended light signals are advantageously reduced. These unintended signals can arise from exciting e.g. impurity molecules, which emit light of a particular wavelength.

In a further preferred embodiment of the invention, the coating layer comprises impregnates modulating the refractive index of the coating layer.

The coating layer further comprises impregnants in the form of e.g. a top layer of the coating layer or as local molecules in the coating layer which are able to modulate the refractive index of the coating layer. Therefore the integral refractive index of the impregnated coating layer can be better matched to the refractive index of the analyte reducing signal losses caused by light scattering at the analyte-impregnated coating layer interface. Therefore the signal to noise ratio of the biosensor device is advantageously further improved. For instance, diethanolamines and triethanolamines can be used as impregnants.

In a further preferred embodiment of the invention, the channels extend through the porous body.

This measure advantageously increases the binding area of the biosensor device resulting in a three-dimensional binding structure for binding the target molecules thereto. Therefore the signal intensity and the sensitivity of the biosensor device is significantly enhanced. Moreover, the biosensor device can be designed in a very small and compact way for providing sufficient signal intensity to be detected.

In a further preferred embodiment of the invention, the substrate and/or the porous body comprises a plurality of defined areas with the capture molecules immobilized in them, wherein said defined areas are separated from one other.

A biosensor device comprising a plurality of defined areas advantageously increases the binding area for the target molecules, whereby the sensitivity of the biosensor device is significantly enhanced. The arrangement of the defined areas can be chosen according to requirements determined by e.g. the detection system or the manufacturing process of the biosensor device. The coating layer can be used to determine the spot size and shape of the defined areas.

In a further preferred embodiment of the invention, capture molecules having different target molecule specificities are immobilized in the defined areas.

In this context, it is to be understood that capture molecules having different target molecules specificities can be immobilized in different defined areas or in one defined area. This measure has the advantage, that the biosensor device can be used for multi-target molecule detection, since different defined areas can show a versatile specificity for various target molecules, whereby the detection of these target molecules can be simultaneously performed. In addition, multi-analyte detection can be performed, as different defined areas having different capture molecule specificity and being separated by the coating layer can be exposed to different analytes. Since the remaining area between the defined areas is covered by the coating layer, cross-signals of adjacent defined areas having capture molecules with different target molecule specificities are prevented. The multi-analyte detection can be simultaneously performed, wherein the defined areas being in contact with the different analytes can be differently shaped. Furthermore, the field of application of one particular biosensor device is advantageously enhanced, since it can be used for multiple detecting of a diversity of target molecules.

Regarding the method of manufacturing the biosensor device in a further preferred embodiment of the invention, the step (d) is carried out before the step (b).

Changing the sequence of the manufacturing steps of the biosensor device causes the functionalization of the porous body with the capture molecules to be performed before applying the coating layer on the remaining area of the substrate, whereby the complete substrate comprises immobilized capture molecules. The first performed functionalization advantageously prevents unintentional immobilization of the capture molecules on the coating layer which could lead to binding events of the target molecules to these capture molecules and thus result in a falsified signal. Moreover, the subsequent, partial covering of the substrate can be advantageously adapted to constraints determined by the user of the biosensor device such as the design and the position of the detection system or requirements respecting the implementation of the biosensor device into other measurement units.

Regarding the method of detecting target molecules in an analyte in a further preferred embodiment of the invention, the target molecules are labeled with label molecules before binding the target molecules to the defined area.

In this context, target molecules and label molecules form complexes which bind to the capture molecules. Labeling the target molecules with label molecules advantageously offers the possibility of inducing a distinct signal which can be detecting by the detection system in a very accurate way. Here, labeling the target molecules is a very fast process by carrying out in a free solution without diffusion limitation. Moreover, performing the step of labeling the target molecules with the label molecules before the step of binding the target molecules, here the complexes of the target molecules and the label molecules, to the capture molecules advantageously shortens the total detecting time of the target molecules in the analyte, wherein the step of labeling the target molecules can be carried out in a very easy and fast way by providing the label molecules in the analyte. Label molecules can be chosen from a variety of molecule types, such as enzymes, radioactive molecules, light emitting molecules, light scattering molecules or magnetic molecules.

In a further preferred embodiment of the invention, the label molecules are optical labels.

This measure has the advantage, that optical labels such as fluorescent, luminescent or phosphorescent light emitting molecules or molecules scattering excitation light can be easily handled, since they generate a distinct signal which is independent of any changes of the chemical conditions of the environment.

In a further preferred embodiment of the invention, the target molecules are excited before detecting the signal.

The advantage here is, that the signal of the biosensor device is only generated by those target molecules or complexes of target molecules and label molecules which are bound to the capture molecules, since the time period between bringing the analyte into contact with the substrate and starting the excitation and readout cycle can be chosen sufficiently long. In this way, the signal to noise ratio and thus the sensitivity of the biosensor device is significantly increased.

In a further preferred embodiment of the invention, the analyte is brought into contact with the substrate of the biosensor device by pumping it across the substrate.

The advantage here is, that a forced movement of the analyte in particular directions across the substrate results in a contact of the analyte with the total defined area, whereby signal losses resulting from an insufficient contact between the analyte, i.e. the target molecules, and the capture molecules are diminished. In particular, pumping the analyte across the porous substrate allows the analyte to penetrate into the functionalized channels of the defined area, whereby the wall surfaces of the channels come into contact with the target molecules. Here, the capillary effect between the analyte and the channels and thus the diffusion limited flow inside the channels is overcome by the forced directed movement of the analyte across the surface of the porous substrate. Furthermore, the pumping cycle increases the residence time of the target molecules at the surface of the defined area, as the analyte can be repeatedly pumped through the channels or forth and back within a housing of the biosensor device. Therefore the probability of specific binding events and thus the detection signal are increased, as the analyte binding kinetics turn more efficient.

Repeatedly pumping the analyte across the surface of the substrate results in a "washing step" of the biosensor device, since unspecific analyte bindings and adsorption of molecules to the binding area are reduced. To further reduce unspecific binding and adsorption a following separate washing solution which is analyte-free can be pumped across the surface of the substrate. These washing steps are preferably performed after the complex-building of the (labeled) target molecules and the capture molecules and before detecting the signal.

In a further preferred embodiment of the invention, the analyte is pumped through the channels of the at least one defined area of the porous body.

In this context, it is to be understood that the analyte can penetrate into and through the channels of the defined area(s) of the porous body. The advantage here is, that the probability of binding events of the target molecules to the capture molecules and thus the sensitivity of the biosensor device is enhanced, since the analyte comes into contact with the capture molecules immobilized on the channel surfaces and the residence time of the target molecules in these channels is increased.

Further advantages will be apparent from the following description and the accompanying drawings. It is to be understood that the afore-mentioned features and those to be explained below are not only applicable in the combinations given, but also in other combinations or in isolation without departing from the scope of the invention.

### BRIEF DESCRIPTION OF THE DRAWINGS

An exemplary embodiment of the invention is illustrated in the drawings and will be described hereinafter with reference thereto. In the drawings:
Fig. 1 is a schematic view of a biosensor device according to the invention; and
Fig. 2 is a top view of a substrate of the biosensor device in Fig. 1.

### DETAILED DESCRIPTION OF THE PRESENT INVENTION

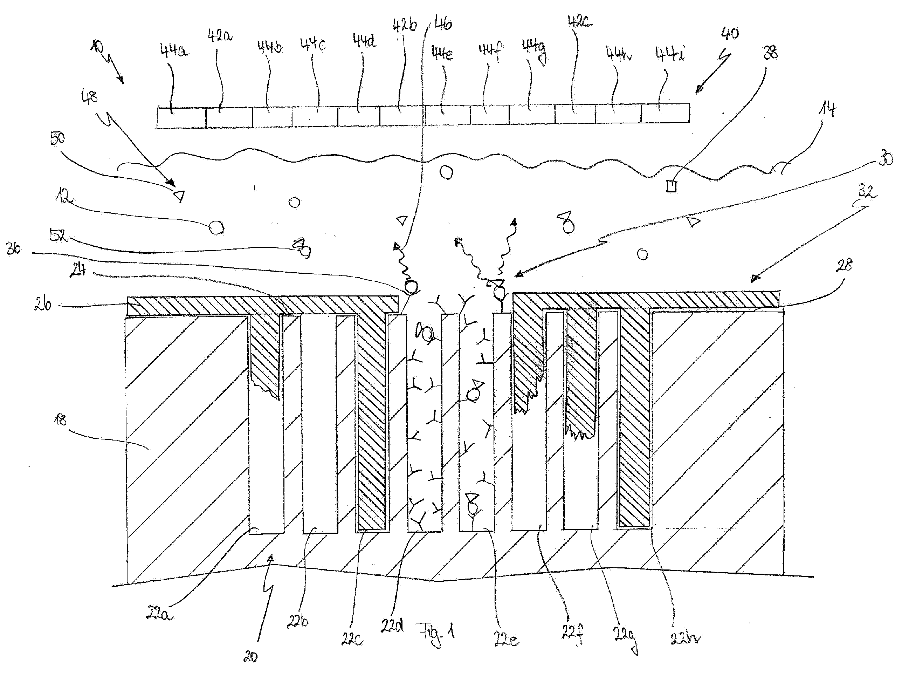

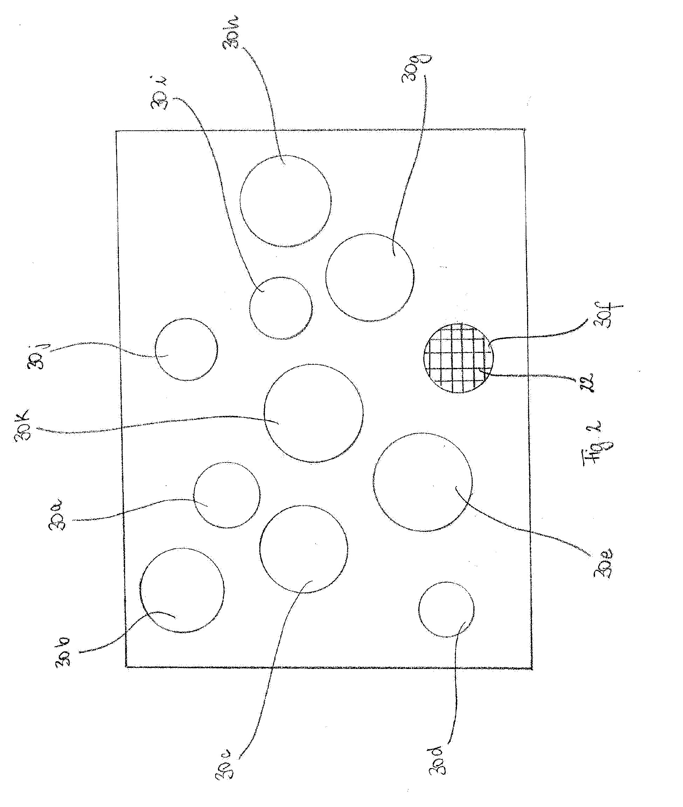

Fig. 1 shows, in a schematic representation, a biosensor device generally labeled with reference numeral 10. Further details of biosensor device 10 are shown in Fig. 2.

The biosensor device 10 is used to detect target molecules 12 in an e.g. water-based analyte 14 e.g. during an immunoassay or a DNA-assay. The sensing principle of the biosensor device 10 is based on binding the target molecules 12 to a binding area 16, eventually exciting the bound target molecules 12 and detecting a signal arising from binding events of the target molecules 12 to a surface of the binding area 16. Preferably, the biosensor device 10 can detect target molecules 12 such as nucleotides like DNA and RNA from humans, bacteria, viruses, fungi, peptides or proteins. However, other target molecules 12 can also be detected by the inventive biosensor device 10.

The biosensor device 10 comprises a substrate 18 which can be brought into contact with the analyte 14 for binding the target molecules 12 thereto. The substrate 18 is designed as rectangular substrate which comprises a porous body 20. In this embodiment the substrate 20 is identical to the porous body 26. However, the porous body 26 can also be designed as a layer which is integrally embedded into the substrate 18 or covers the substrate 18. The material of the porous body 20 is preferably identical to the material of the substrate 18 facilitating the manufacturing process of the biosensor device 10. In this embodiment, the porous substrate 20 is made of a polymeric blotting membrane such as nylon or positively charged nylon, e.g. Nytran® N or Nytran® SuPerCharge of Whatman® with a uniform pore size of 0.20 µm or 0.45 µm.

The particular material of the substrate 18 and the porous body 20 can be chosen with respect to manufacturing requirements such as material costs or detection demands, e.g. an intrinsic affinity of the substrate 18 to the target molecules 12. For instance, the porous body 20 made of sapphire (Al₂O₃), nitrocellulose membranes or Nylon membranes exhibits lower production costs compared to etched silicon or glass fibre bundles. Using nitrocellulose membranes or Nylon further offers the advantage of providing the binding area 16 with an intrinsic affinity for macro-molecules like proteins, DNA or RNA.

The porous body 20 comprises channels 22, here shown eight through-going channels 22 a-h, which are orthogonally arranged with respect to a top surface 24 and a lower surface 25 of the porous body 20. Here, the top surface 24 and the lower surface 25 of the porous body 20 represent the outer surfaces of the porous body 20 between openings of the channels 22 a-h. As shown in Fig. 1, the channels 22 a-h are equally distributed with respect to one another. Although this embodiment represents the ideal case of such a channel structure, it is to be understood that the channels 22 a-h can be irregular in shape, not equidistantly arranged to one another and irregularly distributed over the porous body 20. The channels 22 a-h are formed in such a way that they extend through the porous substrate 18. In case of the porous body 20 being a top layer of the substrate 18 the channels 22 a-h penetrate into the substrate 18. Using the porous body 20 as the binding area 16 for the target molecules 12 offers the advantage of having a three-dimensional surface of the binding area 16, which enhances the signal intensity and thus the sensitivity of the biosensor device 10. Although the particular embodiment of the biosensor device 10 refers to a three-dimensional binding area 16, the inventive biosensor device 10 can make use of two-dimensional binding area 16 or a mixture of a two-dimensional and three-dimensional binding area 16.

A coating layer 26 is partially formed on the top surface 24 and/or the lower surface 25 of the porous body 20 which acts as blocking agent for the target molecules 12. Here, it partially covers the top surface 24 and the lower surface 25 of the porous body 20 and a top surface 28 and a lower surface 29 of the substrate 18. The coating layer 26 is formed around at least one defined area 30 of the porous body 20 which comprises at least one of the channels 22 a-h, here the two channels 22 d, e. A remaining area 32 of the porous body 20 is covered by the coating layer 26. As shown in Fig. 1, the coating layer 26 at least partially extends into the channel 22 a, c, f-h. In this embodiment, the coating layer 26 totally penetrates into the channels 22 c, h, whereas the channels 22 a, f, g are only partially filled, so that wall surfaces 34 of the channels 22 a, c, f-h are at least partially covered with the coating layer 25. The channel 22 b is sealed by the coating layer 26 which does not penetrate into it. Depending on the diameter of one particular channel 22 a-c, f-h the wall surfaces 34 of this channel 22 a-c, f-h can be covered by the coating layer 26, wherein the channel 22 a-c, f-h is not filled up by the material of the coating layer 26.

In general, the coating layer 26 is made of a polymer material such as acrylate or a photo resist. It can also be formed of a copolymer, e.g. acrylate and PMMA or acrylate and a cleavable cross-linker (BAC). The coating layer 26 can be applied by spin coating the material of the coating layer 26 on the total surface 24, 25 of the porous body 20 and eventually on the wall surfaces 34 of the channels 22 a-h and polymerizing the material by a redox catalysis or UV light. The coating layer 26 is removed from the defined area 30 by e.g. spotting a solution with an appropriate pH value or a thiol agent on the surface 24, 25 of the porous body 20, whereby the coating layer 26 is removed from the corresponding surface 24, 25 of the defined area 30 and the associated wall surfaces 30 of the channels 22 d, e. In this way, the coating layer 26 is solution sensitive and/or photosensitive.

Preferably, the coating layer is a polymeric hydrogel based on acrylamide and bis-acrylamide or methacrylate and dimethacrylate, whose polymerisation photoinitiators can be water soluble thioxanthone and benzophenone. In the following, the manufacturing of a particular coating solution is described which is spotted on the surface 24, 25 of the porous body 26, in order to form the coating layer 26 of acrylamide and bis-acylamide. In order to obtain a 25mL coating solution of acrylamide and bis-acrlylamide, 6.665 mL stock solution of acrylamide/bis-acrylamide with a total monomer concentration of 30% and 10 weight % of cross linker, 12.0 g urea and water are mixed with respect to a final volume of 22 mL. For aiding in dissolving the urea, the mixture is warmed to a maximum temperature of 30°C. The mixture is then dissolved to a volume of 25mL and vacuum degassed, in order to remove air bubbles. During vacuum degasing, only a maximum of 10% of the solvent shall be removed. After degasing the final volume has to be rechecked and eventually adapted to 25 mL. In the following, 0.02 mL of TEMED catalyst is added and the solution is mixed thoroughly. By adding 0.08 mL of 10% (Wt./v) ammonium persulphate to the solution the polymerization is initiated, where the polymerization is supported by thoroughly mixing the solution. Within approximately ten minutes the solution shall be spotted to the surfaces 24, 25, 28, 29 of the porous substrate 18 for avoiding the polymerization rendering the solution too viscous. For increasing the period of polymerization the solution can be chilled e.g. in an ice bath, which leads to a retardation of the polymerization. Quenching of the polymerization of the solution spotted on the surfaces 24, 25, 28, 29 of the porous substrate 18 is accomplished by coating a surface of the polymer coating layer 26 with a non-adherent plastic cover sheet such as the GEL-FIX^{™} cover of Serva or by exposing the coating layer 26 to an inert gaseous atmosphere such as argon. Here, the argon causes a displacement of the oxygen atoms of the coating layer 26 which leads to the quenching of the polymerization.

A thickness of the hydrogel coating layer 26 depends on its equilibrium state with the water and preferably varies between 5 nm and 1 mm and further preferably between approximately 1 nm and 10 mm. Above a thickness of approximately 10 mm the hydrogel coating layer 26 turns mechanically unstable.

As the coating layer 26 acts as blocking agent for the target molecules 12, it generally does not offer a binding partner for the target molecules 12 in the sense of free bonds, so that the target molecules 12 are bound to the channels 22 d, e being not covered the coating layer 26. Moreover, the coating layer 26 can even repulse the target molecules 12 based on their intrinsic properties. For instance, in case of a charged coating layer 26, the target molecules 12 which are equally charged are moved away from the coating layer 26. In this way, unspecific bindings of the target molecules 12 to the coating layer 26 are reduced, whereas the probability of specific bindings of the target molecules 12 to the channels 22 d, e of the defined area 30 occurs with an increased binding kinetics and efficiency.

Capture molecules 36 are immobilized in the defined area 30 leading to an enhancement of the target molecule concentration at the binding area 16, whereby the signal intensity and thus the sensitivity of the biosensor device 10 are increased. The capture molecules 36 act as recognition partner for a particular complementary target molecule type, so that only distinct target molecules 12 can bind to the capture molecules 36, whereas other molecules in the analyte 14, e.g. impurity molecules 38 do not bind.

Depending on the sort of the target molecules 12, the capture molecules 36 preferably are single stranded oligonucleotides with lengths between 20 and 30 bases working in a hybridization assay. The capture molecules 36 can also be nucleotides such as DNA or RNA, proteins working in an antibody-antigen-assay, nucleic acids or peptides or proteins. For instance, the capture molecules 36 can be spotted by inkjet printing and immobilized by e.g. UV crosslinking at predefined areas.

Fig. 2 shows a top view of the surface of the binding area 16. This surface shows the eleven defined areas 30 a-k which are not covered by the coating layer 26. Thus only the channels 22 of the defined areas 30 a-k, which are indicated in the defined area 30 f as square lattice, are accessible for the target molecules 12. The defined areas 30 a-k are spaced from one another by the remaining area 32, and they are circularly shaped. However, they can be formed as squares or with any irregular shape and every defined area 30 a-k can show a different spot size and shape. The defined areas 30 a-k are preferably equally distributed and arranged as close as possible to one another for achieving the maximum binding area 16.

The channels 22 of the defined area 30 a-k are functionalized with the capture molecules 36, so that the defined areas 30 a-k provide an enhanced binding area 16 for the target molecules 12. In order to achieve a multi-usage capability of the biosensor device 10, capture molecules 36 having different specificities for different target molecules 12 are immobilized in different defined areas 30 a-k, whereby each defined area 30 a-k then represent a binding area 16 for the distinct target molecules 12. The biosensor device 10 is thus sensitive to various kinds of the target molecules 12 which are simultaneously or subsequently detected during the detection step. It is also possible, that the capture molecules 36 with different target molecule specificities are immobilized in one defined area 30 a-k, whereby the biosensor device 10 can be designed in a very small and compact way. This way of functionalizing the defined area 30 a-k is preferred for target molecules 12 exhibiting a large signal, where a small binding area 16 is sufficient for the signal intensity.

Again referring to Fig. 1, the biosensor device 10 comprises a detection system 40 which is arranged next to the porous body 20 and spaced from the analyte 14 for avoiding an electrical short circuit. In this embodiment, the optical detection system 40 is located next to the top surface 28 of the substrate 18, however, it can also be located next to the lower surface 29 of the substrate 18 or next to both surfaces 28, 29. The detection system 40 is designed as optical detection system comprising three light sources 42 a-c embedded into nine light sensing diodes 44 a-i. The light sources 42 a-c are spaced from one other and equally distributed between the diodes 44 a-i. They are used to excite the surface of the binding area 16, so that bound target molecules 12 generate an optical signal, here fluorescent light 46, which is detected by the diodes 44 a-i. The signal can also be luminescent light, phosphorescent light or any light scattered by the bound target molecules 12.

Signal losses resulting from the light scattering, in particular light reflection, at the substrate-analyte interface are reduced, as the hydrogel coating layer 26 fills the channels 22a-c, f-h with the coating material. Therefore the sensitivity of the biosensor device 10 is enhanced.

For the target molecules 12 not exhibiting intrinsic fluorescent properties or for a distinct modification of the signal with respect to the optimum signal detection capability of the detection system 40, the target molecules 12 can be labeled with label molecules 48, here with optical labels in the form of fluorescent molecules 50. The fluorescent molecules 50 are distributed in the analyte 14 and exhibit an intrinsic affinity for the target molecules 12, so that they bind to the target molecules 12. Furthermore, the concentration of the fluorescent molecules 50 is chosen in such a way, that essentially all target molecules 12 present in the analyte 14 bind to one fluorescent molecule 50 forming a complex 52 of a fluorescent molecule 50 and a target molecule 12. The fluorescent molecules 50 are excited by the light sources 42 a-c and emit the fluorescent light 46 to be detected. The excitation occurs for all fluorescent molecules 50 which are not bound to the target molecules 12, which are complexed in the complexes 52 and which are complexed in the complexes 52 and immobilized to the capture molecules 36. Therefore the immobilization step of the complexes 52 to the capture molecules 36 advantageously results in an enhanced specificity of the biosensor device 10 with respect to the target molecules 12. In addition, immobilizing or fixing the complexes 52 to the defined areas 30 of the porous substrate 18 leads to the possibility to wash off the excess of the label molecules 48. In this context, after the binding process of the labeled target molecules 12 to the capture molecules 36 a separate washing step can be applied which will be described later.

It is also possible to employ other label molecules 48 such as enzymes, radioactive molecules or any molecules which leads to e.g. a visible color change of the analyte. The usage of the label molecules 48 can be adapted to the detection demands of the detection system 40.

In case of a DNA assay, where the target molecules 12 and the capture molecules 36 are DNA molecules and the complementary DNA molecules and hybridize with one another, the label molecules 48 can be oligonucleotides which are labeled with e.g. color molecules, enzymes such as horse radish peroxidase, the fluorescent molecules 50, phosphorescent molecules, luminescent molecules, metal or magnetic particles, in order to facilitate the detection process and increase the sensitivity of the biosensor device 10. In case of an immunoassay, the label molecules 48 can be peptides or proteins.

In general, the label molecules 48 hybridize to the target molecules 12 at a different area than the capture molecules 26, whereby the specificity of the biosensor device 10 is enhanced. In case of DNA molecules and proteins, the label molecules 48 bind to a different sequence and to a different binding site than the capture molecules 36, respectively.

In order to increase the probability of specific binding events of the target molecules 12 or the complexes 52 to the capture molecules 36 immobilized in the defined areas 30 a-k, the analyte 14 can be pumped across the porous body 20. In this way, the biosensor device 10 comprises a flow chamber (not shown) having pumping means causing a directed movement of the analyte 14 through a housing of the flow chamber. The analyte 14 is thus brought into contact with the total surface of the defined areas 30 a-k, whereby it also penetrates into the channels 22 d, e. Preferably, during one pumping cycle the analyte 14 is e.g. completely pumped through the channels 22 d, e of the porous body 20, so that a thickness of the analyte 14 between the optical detection system 40 and the porous substrate 18 is as small as possible. Moreover, the analyte 14 can be pumped back and forth within the flow chamber for increasing the residence time of the target molecules 12 near the binding area 16. As the coating layer 26 covers the not functionalized surface of the porous body 20, the number of pumping cycles can be decreased for achieving an equivalent signal compared to a biosensor device 10 comprising no coating layer 26. Thus the step of detecting the target molecules 12 can be performed within a short period of time. Repeatedly pumping the analyte 14 across the substrate 18 and/or through the channels 22d, e will further decrease the background signal of the biosensor device 10, as during these "washing steps" unspecific bindings of the analyte 14 and adsorption of molecules to the binding area 16 is decreased. Preferably, these washing steps are carried out in a separate washing step after the complex-building of the labeled target molecules 12 and the capture molecules 36 and before exciting the binding are 16 by light. In addition, a following separate washing solution can be pumped across the porous substrate 18 and through the channels 22 d, e to further reduce unspecific binding and adsorption and to wash off the remaining not bound label molecules 48 and the complexes 52 which are not immobilized to the capture molecules 36.

Moreover, a refractive index of the coating layer 26 and its structure in the form of e.g. a distinct grating can be matched to the detection system 40 which will further increase the sensitivity of the biosensor device 10.

The refractive index of the coating layer 26 can be adapted to a refractive index of the analyte 14 in terms of supplying a layer with a refractive index being between the refractive index of the analyte 14 and a refractive index of the substrate 18. Therefore light scattering at the analyte-coating layer and coating layer-substrate interface is reduced and signal losses are avoided. In case of the hydrogel coating layer 26, its refractive index can be modified by changing the ratio of monomer and cross linker co-monomer. It may vary between 1.3 and 1.5, where the refractive index of the analyte 14 is approximately 1.33 and the refractive index of the nylon substrate 18 is approximately 1.56. Preferably, the refractive index of the hydrogel coating layer 26 is approximately 1.49.

Moreover, the coating layer 26 comprises impregnants 54 such as diethanolamides and triethanolamides which are either homogenously distributed in the coating layer 26 or only in defined areas of the coating layer 26. These impregnants 54 further modulate the integral refractive index of the coating layer 26 for reducing signal losses.

The coating layer 26 further comprises polymerizable or non-polymerizable colorants 56 such as benzophenones (hydroxybenzophenones) which act as optical filters for selective wavelengths of light. Therefore unintentional signals such as background signals can be absorbed. Using benzophenones as the colorants 56 is especially advantageous, since they also act as photoinitiators for the polymerization of the hydrogel coating layer 26.

The coating layer 26 can further comprise layers 58 with different refractive indices which act as optical filters or waveguides. These layers 58 can be formed as grating. In this way, further signal losses are reduced, whereby the signal to noise ratio of the biosensor device 10 is further increased.

The biosensor device 10 is manufactured in the following way. First a substrate 18 is provided which comprises the porous body 20 having the channels 22 a-h. After having covered the porous body 20 with the coating layer 26, the coating layer 26 is removed from the defined areas 30 a-k. In case of the afore-mentioned copolymer coating layer 26 the polymer material can be dissolved at the defined area 30 a-k by spotting a solution with a high/low pH or a thiol reducing agent on the top surface 24 and the lower surface 25 of the porous body 20. The particular solution is chosen according to the properties of the material of the coating layer 26. In the further step, the capture molecules 36 are immobilized in the defined area 30 a-k for functionalizing the open channels 22 d, e. This can be performed by spotting the capture molecules 36 on the surfaces 24, 25 of the porous body 20, wherein the capture molecules 36 self-handed distribute over the surfaces 24, 25 of the porous body 20 and bind to the defined areas 30 a-k. The coating layer 26 thus represents a mask on top of the porous body 20 which exhibits holes with the channels 22 d, e being accessible for the target molecules 12. The biosensor device 10 can also be manufactured by first functionalizing the surfaces 24, 25 of the porous body 20 and afterwards putting the coating layer 26 on the porous body 20. By removing the coating layer 26 from the defined areas 30 a-k specific channels 22 d, e are open for the target molecules 12.

## Claims

1. A biosensor device (10) for detecting target molecules (12) in an analyte (14), comprising a substrate (18) which can be brought into contact with the analyte (14) for binding the target molecules (12) thereto, wherein capture molecules (36) for the target molecules (12) are immobilized in at least one defined area (30 a-k) of the substrate (18), wherein the remaining area (32) of the substrate (18) is covered with a coating layer (26) which acts as a blocking agent for the target molecules (12).

2. The biosensor device of claim 1, wherein the substrate (18) comprises a porous body (20) having channels (22 a-h), wherein the defined area (30 a-k) comprises at least one of the channels (22 a-h).

3. The biosensor device of claim 1 or 2, wherein the coating layer (26) is a photo sensitive material.

4. The biosensor device of anyone of claims 1 through 3, wherein the coating layer (26) is made of a polymer.

5. The biosensor device of anyone of claims 1 through 4, wherein the coating layer (26) is made of a photo resist.

6. The biosensor device of anyone of claims 1 through 5, wherein the coating layer (26) is a solution sensitive material.

7. The biosensor of anyone of claims 1 through 6, wherein the substrate (18) and/or the porous body (20) is made of a polymer.

8. The biosensor device of anyone of claims 1 through 7, further comprising a detection system (40) for detecting a signal generated by the target molecules (12) upon binding to the at least one defined area (30 a-k).

9. The biosensor device of claim 8, wherein the detection system (40) is an optical detection system which is arranged next to the coating layer (26).

10. The biosensor device of anyone of claims 1 through 9, wherein a refractive index of the coating layer (26) is adapted to a refractive index of the analyte (14).

11. The biosensor device of anyone of claims 1 through 10, wherein the coating layer (26) comprises colorants (56) acting as optical filters.

12. The biosensor of anyone of claims 1 through 11, wherein the coating layer (26) comprises impregnates (54) modulating the refractive index of the coating layer (26).

13. The biosensor device of anyone of claims 1 through 12, wherein the channels (22 a-h) extend through the porous body (20).

14. The biosensor device of anyone of claims 1 through 13, wherein the substrate and/or the porous body (20) comprises a plurality of defined areas (30 a-k) with the capture molecules (36) immobilized in them, wherein said defined areas (30 a-k) are separated from one other.

15. The biosensor device of claim 14, wherein capture molecules (36) having different target molecule specificities are immobilized in the defined areas (30 a-k).

16. A method of manufacturing a biosensor device (10) for detecting target molecules (12) in an analyte (14), comprising the steps of:
(a) providing a substrate (18),
(b) covering the substrate (18) with a coating layer (26) which acts as a blocking agent for the target molecules (12),
(c) removing the coating layer (26) from at least one defined area (30 a-k) of the substrate (18),
(d) immobilizing capture molecules (36) for the target molecules (12) in the at least one defined area (30 a-k) of the substrate (18).

17. The method of claim 16, wherein the substrate (18) comprises a porous body (20) having channels (22 a-h), wherein the at least one defined area (30a-k) comprises at least one of the channels.

18. The method of claim 16 or 17, wherein the step (d) is carried out before the step (b).

19. The method of anyone of claims 16 to 18, wherein the substrate (18) and/or the porous body (20) is made of a polymer.

20. A method for detecting target molecules (12) in an analyte (14), comprising the steps of
(a) providing a biosensor device (10) of anyone of claims 1 to 15,
(b) binding target molecules (12) to at least one defined area (30 a-k) of a substrate (18) of the biosensor device (10),
(c) detecting a signal generated by the target molecules (12) upon binding to the defined area (30 a-k).

21. The method of claim 20, wherein the substrate (20) comprises a porous body (26) having channels (22a-h), wherein the at least one defined area (30a-h) comprises at least one of the channels (22a-h).

22. The method of claim 20 or 21, wherein the target molecules (12) are labeled with label molecules (48) before binding the target molecules (12) to the defined area (30 a-k).

23. The method of anyone of claims 20 - 22, wherein the target molecules (12) are excited before detecting the signal.

24. The method of anyone of claims 20 through 23 wherein the analyte (14) is brought into contact with a substrate (18) of the biosensor device (10) by pumping it across the substrate (18).

25. The method of anyone of claims 20 through 24, wherein the analyte (14) is pumped through the channels (22 a-h) of the at least one defined area (30 a-k) of the porous body (20).
